# EUROPEAN PATENT APPLICATION

(11) **EP 2 823 814 A1**
(43) Date of publication of application: **14.01.2015**
(21) Application number: 14187905.6
(22) Date of filing: 10.06.2009
(51) Int. Cl.: A61K 31/122, A61K 31/66, A61P 27/02

(54) **Pharmaceutical composition for use in medical and veterinary ophthalmology**

(62) Divisional of application: 09845897.9
(71) Applicant: Mitotech SA, 2661 Luxembourg (LU)
(72) Inventor: Skulachev, Maxim Vladimirovich, 119234 Leninskie Gory (RU)
(74) Representative: Hoffmann Eitle

(57) **Abstract**

The invention relates to pharmaceutics, medicine, in particular to manufacturing and use of pharmaceutical compositions of medicines (ophthalmic preparations) comprising mitochondria-addressed antioxidant and a set of auxiliary substances providing effective treatment for ophtalmological diseases in humans and animals.

## Description

### Field of the invention

The invention relates to pharmaceutics, medicine, in particular to manufacturing and use of pharmaceutical compositions of medicines (ophthalmic preparations) comprising mitochondria-addressed antioxidant and a set of auxiliary substances providing effective treatment for ophtalmological diseases in humans and animals.

### Background of the invention

Natural and synthetic antioxidants are widely used in medical practice for treatment and prophylaxis of eye diseases because oxidative stress is one of the key reasons for the development of different eye pathologies.

Mitochondria-addressed antioxidants that can be reduced and oxidized by the enzymes of the electron transport chain of mitochondria are one of the most effective antioxidants known to date (see Skualchev V.P. (2005), IUBMB Life., 57:305-10; Skulachev V.P. (2007) Biochemistry (Mosc). 72:1385-96; Antonenko Yu.N. et al. (2008), Biochemistry (Mosc)., 73:1273-87).

However, said mitochondrial antioxidants have some peculiarities that complicate their use in practice. The complicated dependence of the antioxidant efficiency on its dose (final concentration in mitochondria) is the main peculiarity. At certain concentrations these substances can act as one of the most powerful prooxidants that can make mitochondria to produce a significant amount of reactive oxygen species (Antonenko Yu.N. et al. (2008), Biochemistry (Mosc).,73:1273-87; Doughan A.K. and Dikalov S.I. (2007), Antioxid Redox Signal. 9:1825-36).

A possibility of treating eye diseases with the aid of mitochondria-addressed antioxidant SkQ1 was shown in several papers, for example in application WO2008048134, and in more detail in article of Neroev et al., (2008) Biochemistry (Mosc)., 73:1317-28. These sources report data on the treatment of some eye diseases with the aid of aqueous solutions of SkQ1. However, at the moment, in *Background of the invention* (that includes said sources) a pharmaceutical composition that comprises mitochondria-addressed antioxidants and is able to treat eye diseases is not disclosed. The unusual physicochemical properties of mitochondria-addressed antioxidants make such composition to be non-obvious. For example, some substances routinely used as part of numerous preparations, - eye drops, accelerate decomposition of SkQ1 (see the experimental examples). The ability of SkQ1 to be irreversibly sorbed at the surface of tubes (vials) containing eye drops was also detected. This leads to drastic and uncontrolled change in the concentration of active ingredient in a pharmaceutical composition that, for said reasons, can fundamentally alter the effectiveness of the composition (up to obtaining the results in practice which are completely the opposite of desirable).

### Description of the invention

The present invention deals with a pharmaceutical composition (eye drops) capable of providing effective use of mitochondrial antioxidants for treatment of eye diseases in humans and animals.

In general, composition of eye drops is a solution comprising the following components **(formula 1**)**:**
Component A1 - mitochondria-addressed antioxidant
Component A2 - pH buffer
Component A3 - concentration stabilizer of mitochondrial antioxidant
Component A4 - prolongator (thickener)
Component A5 - isotonic component
Component A6 - preservative

Eye drops may optionally comprise one or more additional active ingredient (component A7)

Wherein said components are:
**Component A1:** compound comprising targeting moiety, linker group and antioxidant. The general chemical structure of these compounds can be described by a following structure (I): 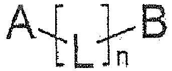 wherein A is effector moiety - antioxidant optionally having a following structure: 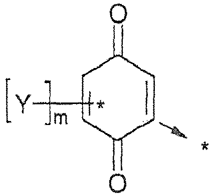 and/or reduced form thereof
   wherein m is an integer from 1 to 3; each Y is independently selected from the group consisting of: lower alkyl, lower alkoxy; or two adjacent Y groups, together with carbon atoms to which they are attached, form a following structure: 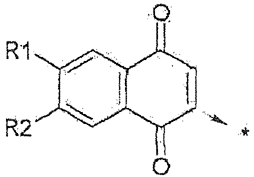 and/or reduced form thereof
   wherein R1 and R2 may be the same or different and are each independently lower alkyl or lower alkoxy;
   L-linker group, comprising:
      a) straight or branched hydrocarbon chain which can be optionally substituted by one or more substituents and optionally contains one or more double or triple bonds;
      b) natural isoprene chain;
   n is integer from 1 to 40, preferably from 2 to 15, particularly preferably from 5 to 11;
   B - targeting group comprising
      a) Skulachev-ion Sk:

         Sk⁺Z"

         where Sk - lipophilic cation, Z - pharmacologically acceptable anion;
      b) charged hydrophobic peptide containing 2-20 amino acid residues,
   as well as solvates, salts, isomers or prodrugs thereof.
   One of compounds preferred for use as component A1 is SkQ1-bromide: 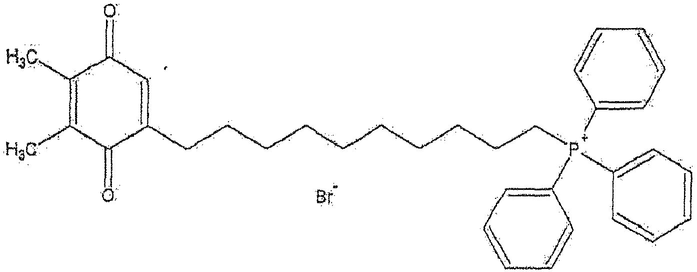 and/or reduced form thereof; along with Br⁻, any other pharmacologically acceptable anion such as Cl⁻, SO₄²⁻ etc. may be selected as anion;
**Component A2:** pH buffer comprising components pharmacologically acceptable in composition of eye drops, it is preferable to use following buffers: phosphate buffer, acetate buffer, borate buffer, carbonate buffer, citrate buffer, Tris buffer, glutamine buffer, epsilon-aminocaproic acid buffer and etc.;
**Component A3: -** concentration stabilizer of mitochondrial antioxidant preventing reversible and irreversible sorption of component A1 on walls of a vial containing said pharmaceutical composition. Irreversible sorption implies both irreversible interaction of component A1 with vial wall material and any chemical transformation of component A1 which occurs as a result of local increase in its concentration near vial walls. Compound which is a lipophilic cation (Skulachev-ion) of specific hydrophilic character, for instance, benzalkonium ion with a pharmacologically acceptable anion (benzalkonium chloride) can be used as concentration stabilizer. It is also possible to use berberine, palmatine, tetraphenylphosphonium, tetrabutyl ammonium or other similar compounds. Vial walls can optionally be chemically modified: concentration stabilizer can be covalently attached to vial wall material. Also, in one embodiment of the present invention, vial wall material is selected in such a way that sorption (or interaction) of component A1 on the walls does not occur (or occurs but insignificantly). In this case, component A3 is not required;
**Component A4: -** prolongator (thickener) is added into eye drops in order to a) increase the viscosity of the solution and thus increase the time the medicine is present on ocular surface, b) stabilize the concentration of active ingredient after instillation (prevention of volume reduction due to moisture loss), c) increase stability of active ingredient during storage. Di-, tri- and poly-saccharides including water-soluble cellulose derivatives, such as methylcellulose, hydroxyethylcellulose, hydroxypropylmethylcellulose and carboxymethylcellulose, sodium chondroitin sulfate, sodium hyaluronate, carboxyvinyl polymer, polyvinyl alcohol, polyvinylpyrrolidone, macrogol etc. can be used as prolongator;
**Component A5: -** isotonic component that endows the composition with osmotic properties suitable for eye drops. Sodium chloride, calcium chloride, glycerol, mannitol, sorbitol, boric acid, glucose, propylene glycol etc. can be used as isotonic component, sodium chloride is the preferred isotonic component;
**Components A6: -** preservatives and optionally stabilizers of additional components of eye drops can be selected from, but not limited to, the following compounds: benzalkonium chloride, benzethonium chloride, chlorhexidine gluconate, chlorbutanol, benzyl alcohol, sodium dehydroacetate, parahydroxybenzoates, sodium edetate, boric acid etc.; sodium bisulfate, sodium thiosulfate, sodium edetate, sodium citrate, ascorbic acid, dibutyl hydroxy toluene etc.

Some compounds can perform functions of several components simultaneously. For instance, in one embodiment of the present invention, 0.1% benzalkonium chloride simultaneously performs function of concentration stabilizer of mitochondrial antioxidant and function of antimicrobial preservative (i.e., it serves as both component A3 and component A6). As part of a pharmaceutical composition of the present invention, component A7 may constitute one and/or more compound selected from the following groups of compounds (given are both compounds themselves and names of preparations and in this case active ingredient of corresponding preparation is meant):
**ANTIOXIDANTS**
   Including N-acetyl cysteine, soluble vitamin E derivatives, ascorbic acid, mannitol, trolox, methylethylpiridinol, taufon etc.
**ANTISEPTICS**
   Furacilinum
   Potassium permanganate (Kaliihypermanganicum)
   Brilliant green (Viride Nitens)
   Iodine solution alcohol, tincture of iodine (Sol. jodi spirituosae, Tinctura iodi 5%)
   Hydrogen peroxide (Sol. Hydrogeniiperoxididiluta)
   Novoimaninum
   Collargolum
   Resorcinol (Resorcinum)
   Zinc sulfate (Zinci sulfas)
**ASTRINGENTS AND CAUTERIES**
   Tannin (Tanninum, Acidum tannicum)
   Xeroform (Xeroformium)
**RESORBENTS**
   Ethylmorphine hydrochloride, dionin (Aethylmorphini hydrochloridum, dioninum) Oxygen (Oxygenium)
   Sodium iodide (Natrium jodidum)
   Potassium iodide (Kalium jodidum)
   Lydasum
**HYPO SENSITIZERS**
   Alergoftal
   Alomide
   Spersallerg
   Calcium chloride (Calcii chloridum)
   Calcium gluconate (Calcii gluconas)
   Diphenhydramine (Dimedrolum)
   Diprazinum, Pipolphen
   Suprastin
   Cusicrom
   Maxidex
   Prenacid
   Lecrolyn, synonym Opticrom, Hay-Crom
**ANTIBIOTICS**
   Benzylpenicillin sodium (potassium) salt (Benzyl-penicillinum-natrium,-kalium)
   Ampicillin trihydrate (Ampicillini trihydras)
   Ampicillin sodium salt (Ampicillinum-natrium)
   Methicillin sodium salt (Methicillinum-natrium)
   Tetracycline (Tetracyclinum)
   Metacycline hydrochloride, rondomycine (Methacyclinum hydrochloridum, Rondomycinum)
   Chloramphenicol (Laevomycetinum)
   Oletetrinum
   Tetraolean, Sigmamycin
   Synthomycin (Synthomycinum)
   Streptomycin sulfate (Streptomycini sulfas)
   Erythromycin (Erythromycinum)
   Gentamicin sulfate, garamycin (Gentamycini sulfas, Garamycin)
   Monomycin (Monomycinum)
   Neomycin (Neomycinum)
   Cefaloridinum, Ceporin
   Lincomycin hydrochloride (Lincomycinum hydrochloridum)
   Nystatin (Nystatinum)
**ANTI-INFLAMMATORY AND ANTIBACTERIAL PREPARATIONS FOR LOCAL USE**
   Garasone
   Dexagentamycin
   Maxitrol
   Tobradex
   Vitabact, Picloxidine
   Fucithalmic
   Colbiocin, Eubetal
   Okacin, Lomefloxacin
   Cipromed, Ciprofloxacin
**SULFANILAMIDE PREPARATIONS**
   Sulfacyl-sodium, sulfacetamide (Sulfacylum-natrium)
   Sulfadimezin (Sulfadimezinum)
   Aethazol (Aethazolum)
   Sulfalen, sulfametopyrazine (Sulfalenum, Kelfizina)
   Sulfapyridazinum
   Sulfadimethoxin, madribon (Sulfadimethoxinum)
**ANTIVIRAL PREPARATIONS**
   Idaxuridin, Kerecid, Oftan-IDU
   Poludanum
   Tebrophen (Tebrophenum)
   Florenal (Florenalum)
   Oxolin (Oxolinum)
   Deoxyribonuclease (Desoxyribonucleasa)
   Zovirax, Aciclovir
   Valtrex, Valaciclovir
   Licopid
**NON-SPECIFIC ANTI-INFLAMMATORY AND REPARATIVE PREPARATIONS**
   Amidopyrine, pyramidon (Amidopyrinum)
   Acetylsalicylic acid, aspirin (Acidumacetylsalicylicum)
   Phenylbutazone (Butadionum)
   Reopyrini
   Indomethacin (Indometacinum)
   Pyrogenalum
   Naclof, Diclo-F, Diclofenac
**LOCAL ANESTHETICS**
   Cocaine hydrochloride (Cocaini hydrocloridum)
   Novocaine (Novocainum)
   Dicain (Dicainum)
   Trimecainum
   Inocainum
**PREPARATIONS FOR SYSTEMIC USE IN OPHTHALMOLOGY**
   Glutamic acid (Acidumglutaminicum)
   Halidor
   Dicynone, etamsylate
   Vinpocetini, vinpocetini-AKRI, cavinton
   Trental, pentoxifylline, pentilin, pentomer
   Solcoseryl
   Taufon, taurine
   Cerebrolysinum
   Emoxypine
**ANTI-CATARACT PREPARATIONS**
   Viceinum
   Vitaiodurolum, vitaphacol
   Qinax
   Cysteine (Cysteinum)
   ATP, adenosine triphosphate (Sol. Natrii adenosintriphosphatis)
**HYPOTENSIVE ANTI-GLAUCOMA MEDICAL PREPARATIONS**
   **Cholinomimetic preparations**
   Pilocarpine hydrochloride (Pilocarpini hydrochloridum)
   Carbachol (Carbacholum)
   Aceclidine (Aceclidinum)
   **Anticholinesterase preparations**
   Armin (Arminum)
   Eserine (Eserinum)
   Proserin (Proserinum)
   Phosphacolum
   Tosmilen, demecarium bromide (Tosmilenum, Demecarii bromidum)
   **Sympathomimetics. Adrenergic preparations**
   Adrenaline (Adrenalinum), eppy, epinephrine, Oftan-dipivefrin
   Dipivalyl epiphrin, dipivephrin
   Clophelin (Clophelinum), isoglaucon
   Apraclonidin, iopidine
   **Antiadrenergic preparations. Beta-adrenergic blockers.**
   Arutimol, timolol maleate, ocumed, optimol, timolol (Timololum), timolol-POS, blocarden, timoptic
   Fotil, fotil-forte
   Proxodolol
   Betaxolol, betoptic
   **Prostaglandins.**
   Latanoprost, xalatan
   **Combination therapies.**
   Timpilo
   Fotil
   Fotil-forte
**CARBONIC ANHYDRASE INHIBITORS**
   Diacarb, acetazolamide
   Trusopt, dorzolamide hydrochloride (Dorzolamide hydrochloridum)
   Rescula, unoprostone
**MYDRIATICS**
   Atropine sulfate (Atropini sulfas)
   Scopolamine hydrobromide (Scopolamini hydrobromidum)
   Homatropine hydrobromide (Homatropini hydrobromidum)
   Platyphylline hydrotartrate (Platiphyllini hydrotartras)
   Adrenaline hydrochloride (Adrenalinihydrochloridum)
   Phetanole (Phetanolum)
   Mydriacyl, tropicamid
   Cyclomed
   Irifrin, phenylephrine
**VITAMINS AND THEIR ANALOGUES**
   Vitamin A, retinol (Vitaminum A, Retinolum)
   Fish oil (Oleum Jecoris Aselli)
   Citral (Citralum)
   Thiamine bromide (Thiamini bromidum), vitamin B₁
   Vitamin B₂, riboflavin, riboflavin-mononucleotide (Riboflavinum, Riboflavinum-mananucleatidum)
**PREPARATIONS THAT RESTORE NATURAL CORNEA MOISTURE**
   Oftagel, carbomer
   Corneregel
   Tears naturale
   Vitacic
   Vidisic
   Lacrysin

### Concentrations of components:

A1 - from 1 to 25000 nM
A2 - concentration sufficient to stabilize pH value in a desired range, preferable concentration is from 0.1 to 1000 mM, most preferably - in the range from 1 to 100 mM, the desired pH range is determined based on the stability of component A1 and the pH optimum for eye drops, and preferably ranging from 4.5 to 8.5, more preferably - from 5 to 8, most preferably - from 7 to 6.
A3 - concentration is determined experimentally and depends on the choice of a specific stabilizer. Molar concentration of stabilizer should exceed the concentration of component A1, the excess should preferably be from 10 to 1000000 times, preferred concentration of stabilizer is in the range from 0.0001% to 1%, particularly preferably - from 0.01% to 0.2%.
A4 - concentration is determined on the basis of the accepted requirements for eye drops depending on selected prolongator, preferably ranging from 0.001 to 1% (w/v).
A5 - concentration that ensures physiologically acceptable osmotic properties of solution (maximally close to osmotic properties of eye fluid).
A6 - concentration of preservative depends on the choice of a particular preservative and can range from a minimum concentration that ensures the stability of the composition during storage, to a maximum concentration that has no harmful effect on eye tissues, in the first place - on cornea.
A7 - concentration of additional active ingredient is determined according to pharmacological properties of a particular compound, the concentration may be reduced based on the enhancement of therapeutic effect by combining additional active ingredient with mitochondria-addressed antioxidant.

In an embodiment of the present invention, a preferred pharmaceutical composition corresponding to **formula 1** is eye drops (per a vial containing 5 ml of eye drops) of the following composition (composition 1):

| | |
|---|---|
| SkQ1 - bromide | about 770 ng |
| Sodium dihydrophosphate | about 4.4 mg |
| Sodium hydrogen phosphate dodecahydrate | about 4.7 mg |
| Hydroxymethyl propyl methylcellulose | about 10 mg |
| Benzalkonium chloride | about 5 micrograms |
| Sodium chloride | about 45 mg |
| Purified water | to 5.0 ml |

wherein SkQ1 - bromide is the following compound with pharmacologically acceptable degree of purity.

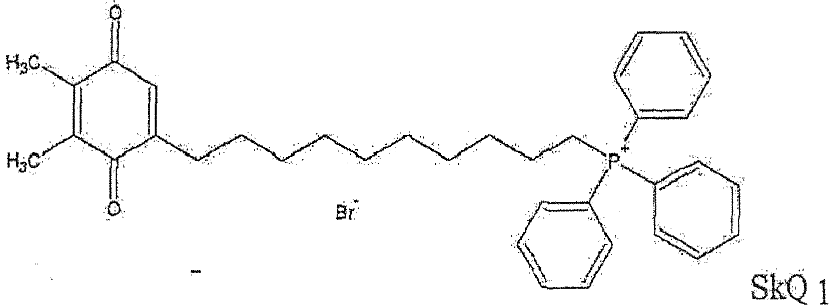

An important aspect of the present invention is the fact that active ingredient of said composition is rather stable, its sorption on the surface of vials is prevented (for confirmation-see the experimental examples). Also, as compared to simple aqueous solution of SkQ1 described in *Background of the invention,* the composition comprises polymer - prolongator chemically compatible with SkQ1. Not all of these polymers, routinely used in composition of eye drops, appeared to be compatible with SkQ1, some polymers destabilized the compound.

Eye drops prepared in accord with said formula turned out to be effective in treating a variety of eye diseases in humans and animals (see the experimental examples). Therefore, an aspect of the invention is use of said eye drops for treatment of patients suffering from different eye pathologies, as well as for prophylaxis of these pathologies.

"Different eye pathologies" comprise but are not limited to: cataract, glaucoma, eye inflammation diseases (including autoimmune), different forms of macular degeneration (MD) and other related symptoms such as atrophic (dry) MD, exudative (wet) MD, age-related maculopathy (ARM), choroidal neovascularization, detached pigment retinal epithelium (PED), atrophy of pigment retinal epithelium (RPE). The term "macular degeneration (MD)" also comprises all eye diseases irrelevant to age-related changes in a human organism such as vitelliform degeneration of Best, Stargardt disease, juvenile macular dystrophy, Behr's disease, Sorsby's dystrophy, Doyne honeycomb retinal dystrophy. "Symptoms related to macular degeneration" comprise but are not limited to: drusen surrounded by white-yellow spots, submacular discoid scar of tissues, choroidal neovascularization, detached pigment retinal epithelium (PED), atrophy of pigment retinal epithelium (RPE), anomalous expansion of choroidal blood vessels, blurred or disturbed vision area, central dead point, pigment anomalies, mixed layer of thin granulation located on the inner side of Bruch's membrane, thickening and lowered permeability of Bruch's membrane.

Said composition can be used for effective prophylaxis or treatment of all forms of macular degeneration and other related syndromes or symptoms, regardless of their causes.

The causes of macular degeneration include, but are not limited to: genetic or physical trauma, diseases such as diabetes, or infections, in particular, bacterial.

In one aspect of the present invention, patient is human.

In another aspect of the present invention, patient is a domestic animal - dog, cat, horse or other. In this case, the composition is used as a veterinary preparation.

### Experimental examples

### 1. Stability of active ingredient in pharmaceutical composition (composition 1).

1. A study of stability of aqueous solutions of SkQ1 (10 mM sodium phosphate, pH 6.5, 0.9% NaCl) in concentration range up to 250 nM shows that addition of 0.01% benzalkonium chloride prevents a decrease in the concentration of active ingredient during storage for 24 hours at room temperature.

| Concentration of benzalkonium chloride,% | Added concentration of SkQ1, nM | Determined concentration of SkQ1 in 24 hours, nM | Relative content of SkQ1, % |
|---|---|---|---|
| 0 (control) | 250 | 104 | 42 |
| 0.01 | 250 | 254 | 102 |
| 0.01 | 180 | 180 | 100 |
| 0.01 | 100 | 97 | 97 |
| 0.01 | 50 | 56 | 112 |

These data show that in the absence of benzalkonium chloride, the concentration of SkQ1 decreases by 58%. This phenomenon is completely prevented by addition of 0.01% benzalkonium chloride, irrespective of the initial concentration of SkQ1.
2. Comparison of the effect of the two most common prolongators in eye drops (methylcellulose - MC and hydroxypropyl methylcellulose - HPMC) on stability of aqueous solutions of SkQ1 (10 mM sodium phosphate, pH 6.5, 0.9% NaCl, 0.01% benzalkonium chloride) shows that only addition of 0.2% HPMC stabilizes the concentration of the active ingredient during storage for 50 days at room temperature.

| Content of prolongators | Relative content of SkQ1 after incubation for 50 days, % |
|---|---|
| 0.2% HPMC | 93 |
| 0.2% MC | 79 |
| control | 83 |

### 2. Clinical cases of use of pharmaceutical composition (composition 1) in veterinary practice

**Veterinary clinical Case 1:**
   Species, breed of animal: dog, cverg schnauzer
   Sex: male
   Age: born in 2005 (4 years old)
   Date of admission: December 4, 2008
   **Case history -** the dog has been blind since November 2008. Since 2007, the dog could not see in the darkness. Now the dog can only keep itself oriented by sound. The owner believes that the animal has been ill for 1 year. The owner drew attention to changes in animal behavior during an evening stroll. The dog does not recognize the mistress in a group of 5-6 people. The dog keeps itself poorly oriented during the daytime, stumbles upon objects. The dog recognizes the owners only by voice.
   In the dark the dog keeps itself oriented by sound.
   Whether the visit to a specialist is primary or secondary - primary visit
   Results of earlier investigations - investigations have not been conducted earlier.
   Diagnosis of disease given earlier - the exact diagnosis was not given.
   Treatment in that period and its efficiency (with a detailed description of the therapy) - treatment was not held.
**Heredity**
   This clinical case is hereditary
**Condition at the time of admission to the clinic**
   The general condition of the animal is satisfactory. The dog does not see. External exam: eyelids have normal volume, palpebral fissure is normal, conjunctiva is pale pink, cornea is shiny, spherical, transparent, moist, contains no blood vessels. Anterior chamber is deep, the reaction of iris to light is missing.

### The preliminary diagnosis and its rationale

1. The diagnosis of underlying disease including severity, form of the disease, the course of disease (acute, subacute, chronic, relapsing, protracted and others), the phase of pathological process activity, the extent (stage) of functional disorders - **Generalized progressive retinal atrophy.**
2. The diagnosis of complications of underlying disease - no.
3. The diagnosis of concomitant diseases (if available) - no.

### Data of laboratory, instrumental methods of research and expert advice

1. Specific tests: labyrinth, reaction to a cotton ball, reaction to light etc. - in the test with a labyrinth, the dog stumbled upon obstacles in darkness and on light, did not react to a cotton ball falling nearby, the reaction of iris to light was absent.
2. Instrumental tests: blood test for the presence of viral and bacterial pathogens (if necessary), autoimmune responses (if necessary), retinography and retino-photography - test for leptospirosis is negative. Retinography was performed. Retino-photography revealed color change t. Lucidum hyper-reflex, thinning of vessels originating from the optic disc (OD), the optic disc is white.
3. Clinical diagnosis and its rationale

Detailed diagnosis of the underlying disease:
1. - name of the disease - generalized progressive retinal atrophy. Stage 2
2. - its clinical, clinico-morphological or pathogenic form - blindness, the surface of t. Lucidum altered, hyper-reflex, small vessels of the retina are absent, thinning of large vessels of the retina. The course of disease - chronic.
3. - extent (stages) of functional disorders or severity of the disease -vision dysfunction in darkness and on light, the animal does not see large stationary or moving objects.

### Prescribed treatment

Pharmaceutical composition (composition 1) in a dose of 1 drop twice a day.

### Record

Data are shown in Table.

| Date | Methods of research | Dynamics of the disease | Prescribed therapy |
|---|---|---|---|
| 04.12.2008 | 1. Labyrinth | Negative | Pharmaceutical composition (composition 1) in a dose of 1 drop once a day. |
| | 2. Reaction to a cotton ball | Negative | |
| | 3. Reaction to light | Negative (mydriasis). | |
| | 4. Retinography | See Appendix | |
| | 5. Retino-photography | Carpet area of t. Lucidum is hyper-reflex, small vessels of retina are absent, thinning of great vessels, OD is white. | |
| | | Right eye: | |
| | | **A-wave - 56.5 micro Volts.** | |
| | Retinogram | **B-wave - 9.5 micro Volts.** | |
| | | **Left eye: A-wave - 9.4 micro Volts, B-wave - 15 micro Volts** | |
| | | | |
| 24.12.2008 | 1. Labyrinth | Negative | |
| | 2. Reaction to a cotton ball | +Negative | |
| | 3. Reaction to light | Positive | |
| | | Fundus of eye is unchanged. | 1 drop once a day. |
| | | The dog no longer stumbled upon objects during the daytime, at night the dog does not see. | |
| | | According to the words of the mistress, the dog began to keep itself oriented better, especially during the daytime. | |
| | | | |
| | 1. Labyrinth | Positive | |
| 13.04.09 | 2. Reaction to a cotton ball | Positive | |
| | 3. Reaction to light | Positive | |
| | | Vision is preserved, the dog sees, keeps itself well-oriented in any time of day. Tests are positive. | |
| | | Vision is preserved. | |
| | | | |
| | Retinography | Right eye: | |
| | | **A-wave - 56.5 micro Volts.** | |
| | | **B-wave - 203.5 micro Volts.** | |
| | | Left eye: **A-wave -45 micro Volts, B-wave - 45.5 micro Volts** | |

### Final epicrisis

A dog, cverg schnauzer breed, with nickname Vintik, in the age of 4 years was admitted to the clinic 04.12.2008. As a result of the acquisition of anamnesis data, clinical examination revealed a generalized progressive retinal atrophy. The dog does not see. Blindness is observed for 1 year, carpet area of t. Lucidum is hyper-reflex, small vessels of retina are absent, thinning of great vessels, OD is white.
Eye drops of composition 1 in a dose of 1 drop twice a day were administered for treatment. After 20 days of the administration of the drops, the dog began to see better during the daytime. After 2 months of the administration of the drops, the dog began to see during the daytime and in the darkness. Tests are positive.
The clinical picture of the fundus of eye is unchanged.

**Retinography indicators: December 4, 2008. Right eye:**
**A-wave - 56.5 micro Volts.**
**B-wave - 9.5 micro Volts.**
Left eye: **A-wave - 9.4 micro Volts,**
**B-wave - 15 micro Volts**
**April 13, 2009 Right eye:**
**A-wave - 56.5 micro Volts.**
**B-wave - 203.5 micro Volts.**
Left eye: **A-wave -45 micro Volts,**
**B-wave - 45.5 micro Volts**

### 3. Clinical cases of use of pharmaceutical composition (composition 1) in combination therapy of eye diseases of domestic animals.

1. A dog, 9 years old, breedless. The dog was admitted to the clinic in late January 2009 with signs of hemorrhagic chorioretinitis and papillitis. At the time of examination the animal has not seen, persistent mydriasis has been detected.
   Since 30th January 2009 treatment was administered:
   Dexamethasone (methylated fluoroprednisolone) 0.1% eye drops.
   1 drop 4 times a day
   After 10 minutes
   Pharmaceutical composition (composition 1), eye drops
   1 drop once a day
   After 10 minutes
   Emoxypine (Methylethylpiridinol) 1%, eye drops
   1 drop 3 times a day.
   On March 16, 2009 the animal began to see that was confirmed by appearance of electroretinogram signal. After that, only pharmaceutical composition (composition 1) (without additional preparations) is used as maintenance therapy.
2. A dog, 11 years old, Labrador.
   The dog was admitted to the clinic in September 2008 with diagnosis of uveodermatological syndrome (endogenous uveitis induced by autoimmune dermatitis). At the time of examination the animal has not seen.
   Treatment.
   1. Cyclomed, eye drops
      1 drop twice a day
      After 10 minutes
   2. Prenacid, eye drops
      1 drop once a day
      After 10 minutes
   3. Indocollyre, eye drops
      1 drop twice a day
      After 10 minutes
   4. Betoptic, eye drops
      1 drop 3 times a day
      After 10 minutes
   5. Pharmaceutical composition (composition 1), eye drops
      1 drop once a day
      After 10 minutes
   6. Emoxypine 1%, eye drops
      1 drop 3 times a day.
      30.01.2009. ERG was performed that revealed that the functional activity of retina is very good, the animal sees. In treatment of uveitis, significant remission of the disease was achieved but since the process is chronic, the full recovery will not happen. Maintenance therapy with the aid of pharmaceutical composition (composition 1) continues.
3. A dog, 7 years old, German Shepherd.
   The dog was admitted to the clinic in September 2008 with diagnosis of endogenous uveitis induced by leptospirosis. Eyesight was preserved.
   Treatment.
   1. Prenacid, eye drops
      1 drop once a day
      After 10 minutes
   2. Indocollyre, eye drops
      1 drop twice a day
      After 10 minutes
   3. Betoptic, eye drops
      1 drop 3 times a day
      After 10 minutes
   4. Pharmaceutical composition (composition 1), eye drops
      1 drop once a day
      After 10 minutes
   5. Emoxypine 1%, eye drops
      1 drop 3 times a day.
      Treatment completed in November 2008 led to full recovery.

## Claims

1. Pharmaceutical composition for treatment or prophylaxis of eye pathologies comprising following components
component A1 - mitochondria-addressed antioxidant
component A2 - pH buffer
component A3 - concentration stabilizer of mitochondrial antioxidant
component A4 - prolongator (thickener)
component A5 - isotonic component
component A6 - preservative
component A7 - additional active ingredient
Wherein said components are:
**Component A1:** mitochondria-addressed antioxidant which is a compound comprising targeting moiety, linker group and antioxidant. The general chemical structure of these compounds can be described by a following structure (I): 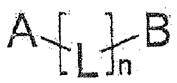 wherein A is effector moiety - antioxidant optionally having a following structure: 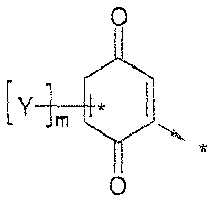 and/or reduced form thereof
wherein m is an integer from 1 to 3; each Y is independently selected from the group consisting of: lower alkyl, lower alkoxy; or two adjacent Y groups, together with carbon atoms to which they are attached, form a following structure: 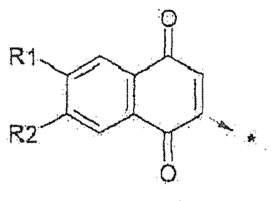 and/or reduced form thereof
wherein R1 and R2 may be the same or different and are each independently lower alkyl or lower alkoxy;
L - linker group, comprising:
a) straight or branched hydrocarbon chain which can be optionally substituted by one or more substituents and optionally contains one or more double or triple bonds;
b) natural isoprene chain;
n is integer from 1 to 40, preferably from 2 to 15, particularly preferably from 5 to 11;
B - targeting group comprising
a) Skulachev-ion Sk:
Sk⁺Z"
where Sk - lipophilic cation, Z - pharmacologically acceptable anion;
b) charged hydrophobic peptide containing 2-20 amino acid residues,
as well as solvates, salts, isomers or prodrugs thereof,
preferably component A1 is SkQ1-bromide: 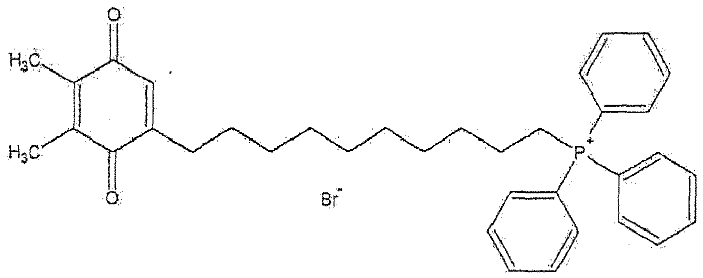 and/or reduced form thereof; along with Br⁻, any other pharmacologically acceptable anion such as Cl⁻, SO₄²⁻ etc. may be selected as anion;
**Component A2:** pH buffer comprising components pharmacologically acceptable in composition of eye drops, it is preferable to use following buffers: phosphate buffer, acetate buffer, borate buffer, carbonate buffer, citrate buffer, Tris buffer, glutamine buffer, epsilon-aminocaproic acid buffer and etc.;
**Component A3: -** concentration stabilizer of mitochondrial antioxidant preventing reversible and irreversible sorption of component A1 on walls of a vial containing said pharmaceutical composition, preferably selected from the following list: pharmacologically acceptable benzalkonium salt (preferably benzalkonium chloride), berberine, palmatine, tetraphenylphosphonium, tetrabutyl ammonium;
**Component A4: -** prolongator (thickener) preferably selected from the following list: di-, tri-and poly-saccharides including water-soluble cellulose derivatives, such as methylcellulose, hydroxyethylcellulose, hydroxypropylmethylcellulose and carboxymethylcellulose, sodium chondroitin sulfate, sodium hyaluronate, carboxyvinyl polymer, polyvinyl alcohol, polyvinylpyrrolidone, macrogol;
**Component A5:** - isotonic component that endows the composition with osmotic properties suitable for eye drops, preferably selected from the following list: sodium chloride, calcium chloride, glycerol, mannitol, sorbitol, boric acid, glucose, propylene glycol etc., sodium chloride is particularly preferred isotonic component;
**Components A6: -** preservative and optionally stabilizer of additional components of eye drops preferably selected from the following compounds: benzalkonium chloride, benzethonium chloride, chlorhexidine gluconate, chlorbutanol, benzyl alcohol, sodium dehydroacetate, parahydroxybenzoates, sodium edetate, boric acid etc.; sodium bisulfate, sodium thiosulfate, sodium edetate, sodium citrate, ascorbic acid, dibutyl hydroxy toluene etc.;
**Component A7 -** additional active ingredient preferably selected from the following list: antioxidants, antiseptics, astringents and cauteries, resorbents, hyposensitizers, antibiotics, anti-inflammatory and antibacterial preparations for local use, sulfanilamide preparations, antiviral preparations, non-specific anti-inflammatory and reparative preparations, local anesthetics, preparations for systemic use in ophthalmology, anti-cataract preparations, hypotensive anti-glaucoma medical preparations, cholinomimetic preparations, anticholinesterase preparations, sympathomimetics. adrenergic preparations, antiadrenergic preparations. beta-adrenergic blockers, prostaglandins, combination therapies, carbonic anhydrase inhibitors, mydriatics, vitamins and their analogues, preparations that restore natural cornea moisture;
provided that the number of components in formula 1 is reduced if compounds simultaneously performing function of several components A1-A7 are used.

2. Pharmaceutical composition according to claim 1 for eye treatment comprising following components (per 5 ml of solution)
| | |
|---|---|
| SkQ1 - bromide | about 770 ng |
| Sodium dihydrophosphate | about 4.4 mg |
| Sodium hydrogen phosphate dodecahydrate | about 4.7 mg |
| Hydroxymethyl propyl methylcellulose | about 10 mg |
| Benzalkonium chloride | about 5 micrograms |
| Sodium chloride | about 45 mg |
| Purified water | to 5.0 ml, |
including composition in which amounts of said components vary within ± 15% due to limitations of accuracy of manufacturing.

3. Pharmaceutical composition according to claim 1 but comprising one or more additional component A7.

4. Composition according to claims 1-3 for treatment and prophylaxis of eye pathologies and diseases.

5. Procedure according to claim 4 when pathology is macular degeneration of retina.

6. Procedure according to claim 4 when pathology is cataract including senile cataract, diabetic cataract.

7. Procedure according to claim 4 when pathology is any pathology of retina (retinopathy), detached retina, retinal vessels, choroid, optic nerve (including optic nerve atrophy), central and peripheral chorioretinal dystrophies, uveitis.

8. Procedure according to claim 4 when pathology is intraocular hemorrhage, traumatic hemorrhage.

9. Procedure according to claim 4 when pathology is inflammatory disease including conjunctivitis, eye ulcers, keratitis, dry keratoconjunctivitis.

10. Procedure according to claim 4 when pathology is glaucoma.

11. Procedure of prophylaxis and/or treatment of eye pathologies of a patient with proviso that this method includes administration of effective amount of pharmaceutical composition described in claims 1-3 to patient requiring this treatment or prophylaxis.

12. Procedure according to claims 4-10 when patient requiring treatment or prophylaxis of disease using pharmaceutical composition is human.

13. Procedure according to claims 4-10 in veterinary medicine when patient requiring treatment or prophylaxis of disease using pharmaceutical composition is animal including domestic animal including dog, cat, horse.

14. Procedure according to claim 11 wherein patient requiring treatment or prophylaxis of disease using pharmaceutical composition is human.

15. Procedure according to claim 11 in veterinary medicine when patient requiring treatment or prophylaxis of disease using pharmaceutical composition is animal including domestic animal including dog, cat, horse.
